# EUROPEAN PATENT APPLICATION

(11) **EP 3 205 719 A1**
(43) Date of publication of application: **16.08.2017**
(21) Application number: 16000374.5
(22) Date of filing: 15.02.2016
(51) Int. Cl.: C12N 5/10, C12P 21/00

(54) **CELL LINES FOR PRODUCING RECOMBINANT GLYCOPROTEINS WITH DI-ANTENNARY N-GLYCANS, METHODS USING THE SAME, AND RECOMBINANT GLYCOPROTEINS**

(71) Applicant: CEVEC Pharmaceuticals GmbH, 51105 Köln (DE)
(72) Inventor: Wissing, Silke, 50670 Köln (DE); Wölfel, Jens, 40764 Langenfeld (DE); Faust, Nicole, 51105 Köln (DE); Kewes, Helmut, 50937 Köln (DE)
(74) Representative: Müller-Boré & Partner Patentanwälte PartG mbB

(57) **Abstract**

The present invention relates to a genetically modified cell line with reduced expression of GnTIVa/b and/or GnTV, a method for the production of glycoproteins having N-glycans with a reduced number of tri- and/or tetra-antennary N-glcyans using said cell line, and respective glycoproteins.

SEQUENCE LISTING

<110>  CEVEC Pharmaceuticals GmbH

<120>  Recombinant glycoproteins with di-antennary N-glycans and cell
       lines for producing the same

<130>  C 4677EU - kl

<160>  3

<170>  PatentIn version 3.5

<210>  1
<211>  741
<212>  PRT
<213>  Homo sapiens

<400>  1

Met Ala Leu Phe Thr Pro Trp Lys Leu Ser Ser Gln Lys Leu Gly Phe
1               5                   10                  15

Phe Leu Val Thr Phe Gly Phe Ile Trp Gly Met Met Leu Leu His Phe
            20                  25                  30

Thr Ile Gln Gln Arg Thr Gln Pro Glu Ser Ser Ser Met Leu Arg Glu
        35                  40                  45

Gln Ile Leu Asp Leu Ser Lys Arg Tyr Ile Lys Ala Leu Ala Glu Glu
    50                  55                  60

Asn Arg Asn Val Val Asp Gly Pro Tyr Ala Gly Val Met Thr Ala Tyr
65                  70                  75                  80

Asp Leu Lys Lys Thr Leu Ala Val Leu Leu Asp Asn Ile Leu Gln Arg
                85                  90                  95

Ile Gly Lys Leu Glu Ser Lys Val Asp Asn Leu Val Val Asn Gly Thr
            100                 105                 110

Gly Thr Asn Ser Thr Asn Ser Thr Thr Ala Val Pro Ser Leu Val Ala
        115                 120                 125

Leu Glu Lys Ile Asn Val Ala Asp Ile Ile Asn Gly Ala Gln Glu Lys
    130                 135                 140

Cys Val Leu Pro Pro Met Asp Gly Tyr Pro His Cys Glu Gly Lys Ile
145                 150                 155                 160

Lys Trp Met Lys Asp Met Trp Arg Ser Asp Pro Cys Tyr Ala Asp Tyr
                165                 170                 175

Gly Val Asp Gly Ser Thr Cys Ser Phe Phe Ile Tyr Leu Ser Glu Val
            180                 185                 190

Glu Asn Trp Cys Pro His Leu Pro Trp Arg Ala Lys Asn Pro Tyr Glu
        195                 200                 205

Glu Ala Asp His Asn Ser Leu Ala Glu Ile Arg Thr Asp Phe Asn Ile
    210                 215                 220

Leu Tyr Ser Met Met Lys Lys His Glu Glu Phe Arg Trp Met Arg Leu
225                 230                 235                 240

Arg Ile Arg Arg Met Ala Asp Ala Trp Ile Gln Ala Ile Lys Ser Leu
                245                 250                 255

Ala Glu Lys Gln Asn Leu Glu Lys Arg Lys Arg Lys Lys Val Leu Val
            260                 265                 270

His Leu Gly Leu Leu Thr Lys Glu Ser Gly Phe Lys Ile Ala Glu Thr
        275                 280                 285

Ala Phe Ser Gly Gly Pro Leu Gly Glu Leu Val Gln Trp Ser Asp Leu
    290                 295                 300

Ile Thr Ser Leu Tyr Leu Leu Gly His Asp Ile Arg Ile Ser Ala Ser
305                 310                 315                 320

Leu Ala Glu Leu Lys Glu Ile Met Lys Lys Val Val Gly Asn Arg Ser
                325                 330                 335

Gly Cys Pro Thr Val Gly Asp Arg Ile Val Glu Leu Ile Tyr Ile Asp
            340                 345                 350

Ile Val Gly Leu Ala Gln Phe Lys Lys Thr Leu Gly Pro Ser Trp Val
        355                 360                 365

His Tyr Gln Cys Met Leu Arg Val Leu Asp Ser Phe Gly Thr Glu Pro
    370                 375                 380

Glu Phe Asn His Ala Asn Tyr Ala Gln Ser Lys Gly His Lys Thr Pro
385                 390                 395                 400

Trp Gly Lys Trp Asn Leu Asn Pro Gln Gln Phe Tyr Thr Met Phe Pro
                405                 410                 415

His Thr Pro Asp Asn Ser Phe Leu Gly Phe Val Val Glu Gln His Leu
            420                 425                 430

Asn Ser Ser Asp Ile His His Ile Asn Glu Ile Lys Arg Gln Asn Gln
        435                 440                 445

Ser Leu Val Tyr Gly Lys Val Asp Ser Phe Trp Lys Asn Lys Lys Ile
    450                 455                 460

Tyr Leu Asp Ile Ile His Thr Tyr Met Glu Val His Ala Thr Val Tyr
465                 470                 475                 480

Gly Ser Ser Thr Lys Asn Ile Pro Ser Tyr Val Lys Asn His Gly Ile
                485                 490                 495

Leu Ser Gly Arg Asp Leu Gln Phe Leu Leu Arg Glu Thr Lys Leu Phe
            500                 505                 510

Val Gly Leu Gly Phe Pro Tyr Glu Gly Pro Ala Pro Leu Glu Ala Ile
        515                 520                 525

Ala Asn Gly Cys Ala Phe Leu Asn Pro Lys Phe Asn Pro Pro Lys Ser
    530                 535                 540

Ser Lys Asn Thr Asp Phe Phe Ile Gly Lys Pro Thr Leu Arg Glu Leu
545                 550                 555                 560

Thr Ser Gln His Pro Tyr Ala Glu Val Phe Ile Gly Arg Pro His Val
                565                 570                 575

Trp Thr Val Asp Leu Asn Asn Gln Glu Glu Val Glu Asp Ala Val Lys
            580                 585                 590

Ala Ile Leu Asn Gln Lys Ile Glu Pro Tyr Met Pro Tyr Glu Phe Thr
        595                 600                 605

Cys Glu Gly Met Leu Gln Arg Ile Asn Ala Phe Ile Glu Lys Gln Asp
    610                 615                 620

Phe Cys His Gly Gln Val Met Trp Pro Pro Leu Ser Ala Leu Gln Val
625                 630                 635                 640

Lys Leu Ala Glu Pro Gly Gln Ser Cys Lys Gln Val Cys Gln Glu Ser
                645                 650                 655

Gln Leu Ile Cys Glu Pro Ser Phe Phe Gln His Leu Asn Lys Asp Lys
            660                 665                 670

Asp Met Leu Lys Tyr Lys Val Thr Cys Gln Ser Ser Glu Leu Ala Lys

675                 680                 685

Asp Ile Leu Val Pro Ser Phe Asp Pro Lys Asn Lys His Cys Val Phe
    690                 695                 700

Gln Gly Asp Leu Leu Leu Phe Ser Cys Ala Gly Ala His Pro Arg His
705                 710                 715                 720

Gln Arg Val Cys Pro Cys Arg Asp Phe Ile Lys Gly Gln Val Ala Leu
                725                 730                 735

Cys Lys Asp Cys Leu
            740

<210>  2
<211>  535
<212>  PRT
<213>  Homo sapiens

<400>  2

Met Arg Leu Arg Asn Gly Thr Val Ala Thr Ala Leu Ala Phe Ile Thr
1               5                   10                  15

Ser Phe Leu Thr Leu Ser Trp Tyr Thr Thr Trp Gln Asn Gly Lys Glu
            20                  25                  30

Lys Leu Ile Ala Tyr Gln Arg Glu Phe Leu Ala Leu Lys Glu Arg Leu
        35                  40                  45

Arg Ile Ala Glu His Arg Ile Ser Gln Arg Ser Ser Glu Leu Asn Thr
    50                  55                  60

Ile Val Gln Gln Phe Lys Arg Val Gly Ala Glu Thr Asn Gly Ser Lys
65                  70                  75                  80

Asp Ala Leu Asn Lys Phe Ser Asp Asn Thr Leu Lys Leu Leu Lys Glu
                85                  90                  95

Leu Thr Ser Lys Lys Ser Leu Gln Val Pro Ser Ile Tyr Tyr His Leu
            100                 105                 110

Pro His Leu Leu Lys Asn Glu Gly Ser Leu Gln Pro Ala Val Gln Ile
        115                 120                 125

Gly Asn Gly Arg Thr Gly Val Ser Ile Val Met Gly Ile Pro Thr Val
    130                 135                 140

Lys Arg Glu Val Lys Ser Tyr Leu Ile Glu Thr Leu His Ser Leu Ile

145                 150                 155                 160

Asp Asn Leu Tyr Pro Glu Glu Lys Leu Asp Cys Val Ile Val Val Phe
                165                 170                 175

Ile Gly Glu Thr Asp Ile Asp Tyr Val His Gly Val Val Ala Asn Leu
            180                 185                 190

Glu Lys Glu Phe Ser Lys Glu Ile Ser Ser Gly Leu Val Glu Val Ile
        195                 200                 205

Ser Pro Pro Glu Ser Tyr Tyr Pro Asp Leu Thr Asn Leu Lys Glu Thr
    210                 215                 220

Phe Gly Asp Ser Lys Glu Arg Val Arg Trp Arg Thr Lys Gln Asn Leu
225                 230                 235                 240

Asp Tyr Cys Phe Leu Met Met Tyr Ala Gln Glu Lys Gly Ile Tyr Tyr
                245                 250                 255

Ile Gln Leu Glu Asp Asp Ile Ile Val Lys Gln Asn Tyr Phe Asn Thr
            260                 265                 270

Ile Lys Asn Phe Ala Leu Gln Leu Ser Ser Glu Glu Trp Met Ile Leu
        275                 280                 285

Glu Phe Ser Gln Leu Gly Phe Ile Gly Lys Met Phe Gln Ala Pro Asp
    290                 295                 300

Leu Thr Leu Ile Val Glu Phe Ile Phe Met Phe Tyr Lys Glu Lys Pro
305                 310                 315                 320

Ile Asp Trp Leu Leu Asp His Ile Leu Trp Val Lys Val Cys Asn Pro
                325                 330                 335

Glu Lys Asp Ala Lys His Cys Asp Arg Gln Lys Ala Asn Leu Arg Ile
            340                 345                 350

Arg Phe Arg Pro Ser Leu Phe Gln His Val Gly Leu His Ser Ser Leu
        355                 360                 365

Ser Gly Lys Ile Gln Lys Leu Thr Asp Lys Asp Tyr Met Lys Pro Leu
    370                 375                 380

Leu Leu Lys Ile His Val Asn Pro Pro Ala Glu Val Ser Thr Ser Leu
385                 390                 395                 400

Lys Val Tyr Gln Gly His Thr Leu Glu Lys Thr Tyr Met Gly Glu Asp
                405                 410                 415

Phe Phe Trp Ala Ile Thr Pro Ile Ala Gly Asp Tyr Ile Leu Phe Lys
            420                 425                 430

Phe Asp Lys Pro Val Asn Val Glu Ser Tyr Leu Phe His Ser Gly Asn
        435                 440                 445

Gln Glu His Pro Gly Asp Ile Leu Leu Asn Thr Thr Val Glu Val Leu
    450                 455                 460

Pro Phe Lys Ser Glu Gly Leu Glu Ile Ser Lys Glu Thr Lys Asp Lys
465                 470                 475                 480

Arg Leu Glu Asp Gly Tyr Phe Arg Ile Gly Lys Phe Glu Asn Gly Val
                485                 490                 495

Ala Glu Gly Met Val Asp Pro Ser Leu Asn Pro Ile Ser Ala Phe Arg
            500                 505                 510

Leu Ser Val Ile Gln Asn Ser Ala Val Trp Ala Ile Leu Asn Glu Ile
        515                 520                 525

His Ile Lys Lys Ala Thr Asn
    530                 535

<210>  3
<211>  548
<212>  PRT
<213>  Homo sapiens

<400>  3

Met Arg Leu Arg Asn Gly Thr Phe Leu Thr Leu Leu Leu Phe Cys Leu
1               5                   10                  15

Cys Ala Phe Leu Ser Leu Ser Trp Tyr Ala Ala Leu Ser Gly Gln Lys
            20                  25                  30

Gly Asp Val Val Asp Val Tyr Gln Arg Glu Phe Leu Ala Leu Arg Asp
        35                  40                  45

Arg Leu His Ala Ala Glu Gln Glu Ser Leu Lys Arg Ser Lys Glu Leu
    50                  55                  60

Asn Leu Val Leu Asp Glu Ile Lys Arg Ala Val Ser Glu Arg Gln Ala
65                  70                  75                  80

Leu Arg Asp Gly Asp Gly Asn Arg Thr Trp Gly Arg Leu Thr Glu Asp
                85                  90                  95

Pro Arg Leu Lys Pro Trp Asn Gly Ser His Arg His Val Leu His Leu
            100                 105                 110

Pro Thr Val Phe His His Leu Pro His Leu Leu Ala Lys Glu Ser Ser
        115                 120                 125

Leu Gln Pro Ala Val Arg Val Gly Gln Gly Arg Thr Gly Val Ser Val
    130                 135                 140

Val Met Gly Ile Pro Ser Val Arg Arg Glu Val His Ser Tyr Leu Thr
145                 150                 155                 160

Asp Thr Leu His Ser Leu Ile Ser Glu Leu Ser Pro Gln Glu Lys Glu
                165                 170                 175

Asp Ser Val Ile Val Val Leu Ile Ala Glu Thr Asp Ser Gln Tyr Thr
            180                 185                 190

Ser Ala Val Thr Glu Asn Ile Lys Ala Leu Phe Pro Thr Glu Ile His
        195                 200                 205

Ser Gly Leu Leu Glu Val Ile Ser Pro Ser Pro His Phe Tyr Pro Asp
    210                 215                 220

Phe Ser Arg Leu Arg Glu Ser Phe Gly Asp Pro Lys Glu Arg Val Arg
225                 230                 235                 240

Trp Arg Thr Lys Gln Asn Leu Asp Tyr Cys Phe Leu Met Met Tyr Ala
                245                 250                 255

Gln Ser Lys Gly Ile Tyr Tyr Val Gln Leu Glu Asp Asp Ile Val Ala
            260                 265                 270

Lys Pro Asn Tyr Leu Ser Thr Met Lys Asn Phe Ala Leu Gln Gln Pro
        275                 280                 285

Ser Glu Asp Trp Met Ile Leu Glu Phe Ser Gln Leu Gly Phe Ile Gly
    290                 295                 300

Lys Met Phe Lys Ser Leu Asp Leu Ser Leu Ile Val Glu Phe Ile Leu
305                 310                 315                 320

Met Phe Tyr Arg Asp Lys Pro Ile Asp Trp Leu Leu Asp His Ile Leu
                325                 330                 335

Trp Val Lys Val Cys Asn Pro Glu Lys Asp Ala Lys His Cys Asp Arg
            340                 345                 350

Gln Lys Ala Asn Leu Arg Ile Arg Phe Lys Pro Ser Leu Phe Gln His
        355                 360                 365

Val Gly Thr His Ser Ser Leu Ala Gly Lys Ile Gln Lys Leu Lys Asp
    370                 375                 380

Lys Asp Phe Gly Lys Gln Ala Leu Arg Lys Glu His Val Asn Pro Pro
385                 390                 395                 400

Ala Glu Val Ser Thr Ser Leu Lys Thr Tyr Gln His Phe Thr Leu Glu
                405                 410                 415

Lys Ala Tyr Leu Arg Glu Asp Phe Phe Trp Ala Phe Thr Pro Ala Ala
            420                 425                 430

Gly Asp Phe Ile Arg Phe Arg Phe Phe Gln Pro Leu Arg Leu Glu Arg
        435                 440                 445

Phe Phe Phe Arg Ser Gly Asn Ile Glu His Pro Glu Asp Lys Leu Phe
    450                 455                 460

Asn Thr Ser Val Glu Val Leu Pro Phe Asp Asn Pro Gln Ser Asp Lys
465                 470                 475                 480

Glu Ala Leu Gln Glu Gly Arg Thr Ala Thr Leu Arg Tyr Pro Arg Ser
                485                 490                 495

Pro Asp Gly Tyr Leu Gln Ile Gly Ser Phe Tyr Lys Gly Val Ala Glu
            500                 505                 510

Gly Glu Val Asp Pro Ala Phe Gly Pro Leu Glu Ala Leu Arg Leu Ser
        515                 520                 525

Ile Gln Thr Asp Ser Pro Val Trp Val Ile Leu Ser Glu Ile Phe Leu
    530                 535                 540

Lys Lys Ala Asp
545

## Description

The present invention relates to a genetically modified cell line with reduced expression of GnTlVa/b and/or GnTV, a method for the production of glycoproteins having N-glycans with a reduced number of tri- and/or tetra-antennary N-glcyans using said cell line, and respective glycoproteins.

The development of therapeutic proteins has accelerated immensely over the past years. However, in particular for complexly glycosylated proteins, it can be difficult to obtain proteins with favorable glycosylation patterns. This often leads to suboptimal pharmacological properties such as *e.g.* reduced serum half-life or increased immunogenicity.

One difficulty in producing recombinant glycoproteins is the potential heterogeneity of the product due to variations in post-translational modifications, in particular of glyco-structures. This can lead to inconsistencies in product quality between different production batches.

Glycosylation is the most common post-translational modification. Nearly all biopharmaceuticals need to be correctly glycosylated in order to display the optimal therapeutic effect. In general, glycosylation refers to the covalent attachment of sugars to the protein surface, wherein the sugars are either linked to asparagine residues resulting in N-linked glycans (N-glycans) or serine or threonine residues resulting in O-linked glycans (O-glycans). The glycosylation pattern can be very diverse between different molecules as the attached glycans can be different in monosaccharide order, branching pattern, and length (micro-heterogeneity). Additionally, not all glycosylation sites are necessarily fully occupied (macro-heterogeneity).

Glycosylation influences *e.g.* the solubility of proteins, their resistance to proteolysis, and their binding behavior to other proteins or protein receptors such as *e.g.* the ASGPR (asialoglycoprotein receptor) and, therefore, influences the half-life of the glycoprotein in the plasma.

For small size proteins under about 50 kDa, clearance occurs mainly via the kidney. Beside the size of a protein, also the protein surface charge has influence on renal clearance, as the filtration of highly charged proteins in the kidney is diminished. For large size proteins, clearance occurs mainly in the liver through specific and/or unspecific hepatic uptake. An example for a specific uptake mediator is the ASGPR, which binds specifically to non-sialylated N-linked glycoproteins with a terminal galactose. Due to the action of this receptor, glycoproteins with terminal sialic acids (N-acetylneuraminic acid; NeuAc) covering the adjacent carbohydrate, galactose, have an up to 100-fold increased half-life as compared to their non-sialylated counterparts with terminal galactose residues on their N-linked glycans. Other receptors bind specifically to mannose, N-acetyl-glucosamine, or fucose and clear glycoproteins with these terminal sugars from the system.

From this perspective, a native or nearly native N-glycosylation pattern including a high degree of terminal sialylation is crucial for therapeutic proteins since it determines the pharmacokinetic properties of the therapeutic protein. In addition, terminal sialic acids with the proper linkage on glycoproteins reduce the immunogenicity of glycoproteins.

The foremost strategy to obtain nearly native glycostructures and a high degree of sialylation is to produce the recombinant proteins in mammalian cell lines capable of linking mammalian-like glycostructures to proteins, *e.g.* CHO cells (Chinese hamster ovary cells). However, non-human mammalian cell lines often lack the enzyme needed to catalyze 2,6-linkage of sialic acids and, therefore, can only catalyze 2,3-linkage. Moreover, in addition to sialic acid, they often also link N-glycolylneuraminic acid (NeuGc) to glycans, a sugar not synthesized in humans and therefore immunogenic in humans when injected. Thus, a better strategy would be to generate therapeutic proteins from human cell lines like CAP cells (derived from human amniocytes) or HEK293 cells (derived from human embryo kidney cells). However, sialylation of therapeutic proteins secreted from human cell lines during fermentation is often incomplete.

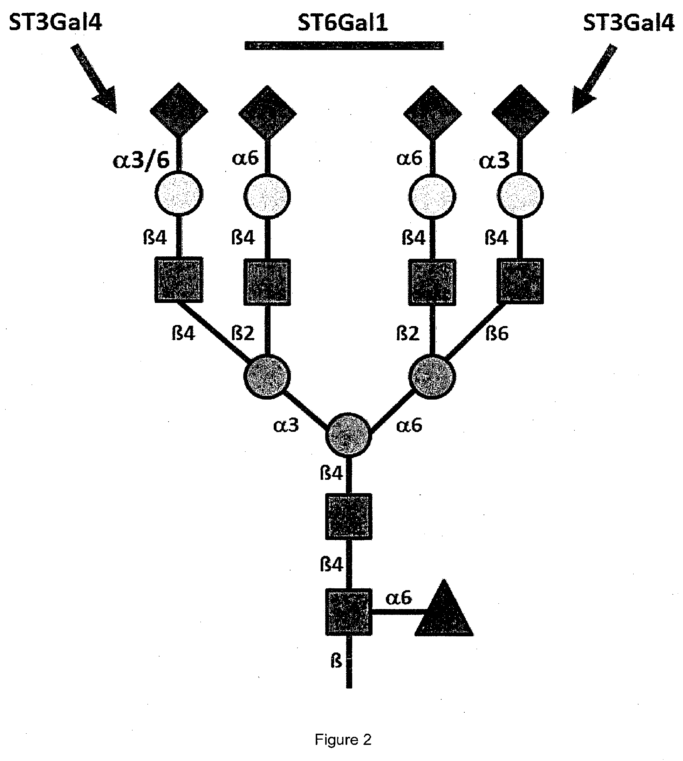

Accordingly, additional expression of sialyltransferases, *e.g.* β-galactoside α-2,6-sialyltransferase 1 (ST6Gal1) or β-galactoside α-2,3-sialyltransferase 4 (ST3Gal4), in mammalian cells could be advantageous to increase sialylation and consequently serum half-life of therapeutic proteins. Interestingly, human serum proteins are mostly 2,6-sialylated, and normally only the additional branches in tri- and tetra-antennary N-glycans are 2,3-sialylated. Reasons for this were revealed in *in vitro* studies with bovine ST6Gal1 enzyme. In particular, it could be shown that this sialyltransferase adds NeuAc very inefficiently to the third and fourth branch of N-glycans, in particular to the Gal*ß*1→4GlcNAc*ß*1→6Manα1→6 branch (Figure 2). In line with these studies it was shown that overexpression of ST6Gal1 results in a lower degree of sialylation of complex N-glycans as compared to expression of ST3Gal4.

Reasons for this are that the third and fourth antennae of a tetra-antennary N-glycan are characterized by different glycosidic linkage and are sterically different. Therefore, they show differential substrate availability to ST3Gal4 and ST6Gal1 enzymes. Accordingly, the Gal*ß*1→4GlcNAc*ß*1→2Manα1→3 and Gal*ß*1→4GlcNAc*ß*1→2Manα1→6 arms are preferentially sialylated by 2,6-linked sialic acid while the Gal*ß*1→4GlcNAc*ß*1→4Manα→3 and Gal*ß*1→4GlcNAc*ß*1→6Manα1→6 arms are preferred targets for 2,3-sialylation.

Therapeutic proteins secreted from mammalian cell lines during fermentation mostly display a higher degree of tri and tetra-antennary N-glycan structures as compared to their counterparts purified from human serum plasma. In line with the studies described above, additional overexpression of ST6Gal1 does not result in completely sialylated therapeutic proteins. Only overexpression of ST3Gal4 does, but in that case the sialylation linkage differs from the plasma-derived proteins. In order to recombinantly produce therapeutic proteins that are highly similar to their naturally occurring counterparts as they are found circulating in the human body, it can thus be necessary to modify the antennarity of their N-linked glycans and optionally also the linkage type of the terminal sialic acids.

α1-Antitrypsin (AAT) is a protease inhibitor belonging to the serpin superfamily. AAT is a potent inhibitor of serine proteases, in particular neutrophil elastase. AAT is a 52 kDa glycoprotein carrying 3 N-glycosylation sites, namely Asn46, 83 and 247, which mainly carry di-antennary (Asn46, 83, and 247) and tri-antennary N-glycans (Asn83). AAT purified from human serum contains α-2,6-linked neuraminic acids at the di- and tri-antennary N-glycans, and some tri-antennary N-glycans contain additionally one single α-2,3-neuraminic acid per N-glycan. The amount of tri-antennary N-glycans in different human plasma derived AAT samples measured by HPLC averages 12% (+/- 0,4%), tetra-antennary N-glycans could not be detected.

Expression of human recombinant AAT in a broad range of mammalian cells has been shown to result in rhAAT (recombinant human AAT) with increased amounts of tri-antennary N-glycans, and even tetra-antennary N-glycan structures to an extent of 10% and more. This is in strong contrast to the human serum derived counterpart. Moreover, in order to achieve full sialylation of all N-glycan branches in such proteins, additional expression of the ST3Gal4 sialyltransferase is necessary, as due to the low affinity to core ß1→4 and core ß1→6 branches of the tri- and tetra-antennary N-glycans, expression of ST6Gal1 sialyltransferase is not efficient enough, resulting in recombinant proteins which not only differ from the plasma protein regarding their N-glycan antennarity but also regarding the sialylation linkage.

Therefore, the technical problem underlying the present invention is the provision of recombinant glycoproteins having homogenous post-translational modifications with primarily di-antennary N-glycans, as well as cell lines that are capable of recombinantly producing such proteins.

The solution to the above technical problem is achieved by the embodiments characterized in the claims.

In particular, in a first aspect, the present invention relates to a cell line that is genetically modified to reduce the expression of at least one of GnTIVa/b and GnTV.

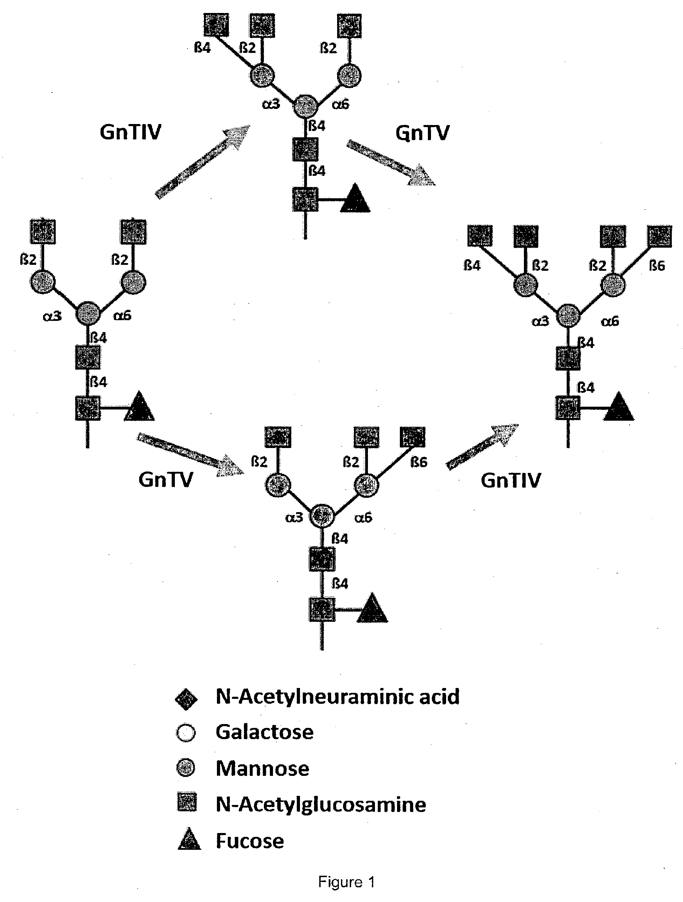

GnTIVa and GnTlVb are glycosyltransferases that participate in the transfer of N-acetylglucosamine (GlcNAc) to the core mannose residues of N-linked glycans. They are isoenzymes that are often equally expressed. They catalyze the formation of the GlcNAc ß1-4 branch on the GlcNAc ß1-2 Man α 1-3 arm of the core structure of N-linked glycans. GnTV catalyzes the addition of GlcNAc in ß 1-6 linkage to the α-linked mannose of di-antennary N-linked oligosaccharides. Both enzymes (GnTlVa/b and GnTV) are essential for the production of tetra-antennary N-linked sugar chains (Figure 1).

In this context, in cell lines wherein GnTIVa and GnTlVb are more or less equally expressed, preferably the expression of both is reduced. However, in cell lines that predominantly express one of GnTIVa and GnTlVb, such as CAP cells expressing predominantly GnTlVb, reduction of the expression of the predominantly expressed isoenzyme may be sufficient.

According to the present invention, the cell lines are genetically modified to reduce the expression of at least one of GnTIVa (α-1,3-mannosyl-glycoprotein 4-β-N-acetylglucosaminyltransferase A; SEQ ID NO: 2), GnTlVb (α-1,3-mannosyl-glycoprotein 4-β-N-acetylglucosaminyltransferase B; SEQ ID NO: 3), and GnTV (α-1,6-mannosylglycoprotein 6-β-N-acetylglucosaminyltransferase A; SEQ ID NO: 1). In preferred embodiments, the expression of GnTlVa/b and GnTV is reduced.

In this context, the term "cell line that is genetically modified to reduce the expression of at least one of GnTIVa/b and GnTV" as used herein indicates that upon genetic modification, the individual cells of the cell line display lower expression levels and, thus, lower activities of the respective proteins than they did before the genetic modification.

The terms "reduced expression" or "reduced activity" as used herein refer to a reduction of protein expression or protein activity by at least 50%, preferably at least 60%, 70%, 75%, 80%, 82%, 84%, 86%, 88%, 90%, 91%, 92%, 93%, 94%, 95%, 95.5%, 96%, 96.5%, 97%, 97.5%, 98%, 98.25%, 98.5%, 98.75%, 99%, 99.25%, 99.5%, 99.6%, 99.7%, 99.8%, or 99.9%.

Genetic modifications that allow the reduction of the expression of a given protein are not particularly limited and are known in the art. In a particular example, the cell line comprises an endogenous GnTlVa/b or GnTV gene, such as *e.g.* human cell lines. In such cases, the cells can be genetically modified by deletion or insertion of nucleotides into the genome of the cells in a position suitable to cause knock-out of said nucleic acid. This can be done by non-homologous end joining or homologous recombination using TALENS, Zn-finger proteins, CRISPR-CAS9, or other methods known in the art. Accordingly, in preferred embodiments, the expression of GnTlVa/b and/or GnTV is completely abolished, preferably by disruption of the respective genes. Reduced expression of a given protein can also be achieved by reduction of the levels of corresponding mRNA, *e.g.* the cells can be genetically modified in order to express RNAi targeting the mRNA of GnTIVa/b and/or GnTV. Respective methods are not particularly limited and are known in the art.

In preferred embodiments, the cell line is selected from the group consisting of insect cell lines, avian cell lines, and mammalian cell lines, wherein mammalian cell lines, in particular human cell lines are particularly preferred. In specific embodiments, the cell line is derived from a cell line, selected from the group consisting of Muscovy Duck cells (AGE.CR^{®}), African green monkey kidney epithelial cells (Vero), Madin Darby canine kidney cells (MDCK), baby hamster kidney cells (BHK), Chinese hamster ovary (CHO) cells, human hepatocarcinoma cell lines (HepG2, Huh7), human embryonic kidney 293 (HEK293) cells, human neuronal precursor cells (AGE1.HN^{®}, NC5T11), human embryonic retinoblast cells (Per.C6), myeloma cell lines (HMCLs, MM.1, U266, RPMI8226), CML tumor cell lines (NM, NM-F9), hybrid HEK293 and lymphoma cell (HKB11), and human amniocytes-derived CAP cells. In this context, CAP cells are particularly preferred.

CAP cells are permanent amniocytic cell lines comprising a nucleic acid encoding the gene products of adenovirus, in particular adenovirus type 5 (Ad5), E1A and E1 B regions. CAP cells are derived from primary human amniocytes that are transformed with a nucleic acid encoding Ad5 E1A and E1B.

Accordingly, in a preferred embodiment, the cell lines according to the present invention can be derived from human primary amniocytes comprising at least one nucleic acid encoding the gene products of the adenoviral E1 and pIX region, preferably E1 and pIX region of adenovirus type 5 (Ad5), more preferably from nucleotides 505 to 4079, in which preferably E1A is under the control of the murine phosphoglycerate kinase (pgk) promoter, while E1 B and pIX expression is controlled from their natural promoters. The E1 B downstream intron, splice acceptor and polyA signal are preferably replaced by corresponding motifs from SV40.

In preferred embodiments, the cell line is further genetically modified to overexpress β-galactoside α-2,6-sialyltransferase 1 (ST6Gal1) and/or α-2,3-sialyltransferase 4 (ST3Gal4).

In this context, the term "overexpressed" either refers to protein expression in cells that prior to genetic modification did not express the respective protein at all, or to an increase in endogenous expression of at least 1.2-fold, preferably at least 1.4-fold, 1.6-fold, 1.8-fold, 2-fold or more.

Methods for the expression/overexpression of a given protein in a cell are not particularly limited and are known in the art. Suitable genes encoding ST3Gal4 and suitable genes encoding ST6Gal1 are not particularly limited and include any genes from any origin that encode a protein having the respective activity, wherein mammalian, in particular human genes are particularly preferred.

In a further aspect, the present invention relates to methods for the expression of recombinant glycoproteins of interest in a cell, said glycoproteins having N-glycans with a significantly increased proportion of di-antennary N-glycans when compared to the same recombinant glycoprotein expressed in a cell line with non-reduced expression of GnTIVa/b and/or GnTV, said methods comprising the step of genetically modifying said cell to reduce the expression of GnTlVa/b and /or GnTV in said cell, and optionally to overexpress ST6Gal1 and/or ST3Gal4.

In particular embodiments, said methods comprise the steps of:
(a) providing a cell line according to the present invention;
(b) expressing the glycoprotein of interest in said cell line; and
(c) isolating the glycoprotein from the cell or the cell culture supernatant.

Means for recombinantly expressing glycoproteins in a given cell line, as well as for isolating said glycoproteins from the cell or the cell culture supernatant, are not particularly limited and are known in the art.

In these aspects, all of the definitions and preferred and/or specific embodiments described below for the recombinant glycoproteins of the present invention, and described above for the cell lines of the present invention apply in an analogous manner where applicable.

In a further aspect, the present invention relates to a recombinant glycoprotein that has been expressed in a genetically modified cell line of the present invention, having N-glycans with a significantly increased proportion of di-antennary N-glycans when compared to the same recombinant glycoprotein expressed in a cell line with non-reduced expression of GnTlVa/b and/or GnTV.

As used herein, the term "recombinant glycoprotein" indicates that the respective glycoproteins are biotechnologically produced in genetically modified or cells.

According to the present invention, the proportion of di-antennary N-glycans in the glycoproteins is preferably increased at least 1.2-fold, more preferably at least 1.4-fold, 1.6-fold, 1.8-fold, 2-fold or more, when compared to the same recombinant glycoprotein expressed in a cell line with non-reduced expression of GnTIVa/b and/or GnTV.

In related preferred embodiments, the proportion of tetra-antennary N-glycans in the glycoproteins is significantly reduced when compared to the same recombinant glycoprotein expressed in a cell line with non-reduced expression of GnTIVa/b and/or GnTV, Preferably, said proportion is reduced at least 2-fold, more preferably at least 5-fold, at least 10-fold, at least 100-fold (corresponding to a reduction by at least 50%, more preferably at least 80%, at least 90%, at least 99%) or more, wherein any remaining N-glycans besides di-antennary N-glycans, if present at all, are tri-antennary N-glycans.

In preferred embodiments, at least 80% of the antennae of the N-glycans of the recombinant glycoproteins of the present invention are terminated by 2,6-linked sialic acid. More preferably, at least 85%, 90%, 92%, 93%, 94%, 95%, 95.5%, 96%, 96.5%, 97%, 97.5%, 98%, 98.25%, 98.5%, 98.75%, 99%, 99.25%, 99.5%, 99.6%, 99.7%, 99.8%, or 99.9% of the N-glycans are terminated by 2,6-linked sialic acid.

Respective glycoproteins can be produced by recombinant expression in suitable organisms or cells, wherein concurrently with expression of the glycoproteins, and reduction of the expression of GnTIVa/b and/or GnTV, ST6Gal1 and/or ST3Gal4 are overexpressed, *e.g.* as indicated above. Preferably, the glycoproteins of the present invention are expressed in a cell line of the present invention as defined above.

The N-glycans of the recombinant glycoproteins of the present invention are characterized by a reduced micro-heterogeneity, *i.e.,* a reduced diversity of N-glycan structure and composition within a given glycoprotein preparation. Respective recombinant glycoproteins can be produced as described herein, e.g. by reducing the expression level and, this, activity of GnTIVa/b and/or GnTV, and optionally by additional overexpression of ST3Gal4 and/or ST6Gal1. Preferably, said glycoproteins are produced in a cell line according to the present invention as described herein.

Specific glycoproteins according to the present invention are not particularly limited, provided that said glycoproteins have N-glycans. Preferably, said glycoproteins are mammalian, more preferably human glycoproteins. Glycoproteins can be selected from the group consisting of growth factors, peptide hormones, cytokines, enzymes, antibodies, antibody fragments, blood clotting factors, and protease inhibitors. However, in a particular embodiment, said glycoproteins are not antibodies or antibody fragments, *i.e.,* the glycoproteins are selected from the group consisting of growth factors, peptide hormones, cytokines, enzymes, blood clotting factors, and protease inhibitors.

Preferably, the glycoprotein is selected from the group consisting of α1-antitrypsin (AAT), hepatocyte growth factor (HGF), erythropoietin (EPO), Factor VII (FVII), Factor VIII (FVIII), Factor IX (FIX), von-Willebrand-Factor (vWF), and C1 esterase inhibitor (C1-inhibitor; C1 Inh), wherein C1 Inh is particularly preferred.

In a further aspect, the present invention relates to a method for the generation of a cell line for the recombinant expression of glycoproteins according to the present invention, comprising steps of genetically modifying a cell line to reduce the expression of at least one of GnTIVa/b and GnTV, and optionally to overexpress ST6Gal1 and/or ST3Gal4.

In further aspects, the present invention relates to methods for decreasing the micro-heterogeneity of N-glycans of recombinant glycoproteins produced in a cell, said method comprising the step of genetically modifying said cell to reduce the expression of GnTIVa/b and /or GnTV in said cell, and optionally to overexpress ST6Gal1 and/or ST3Gal4.

In particular embodiments, said methods comprise the steps of:
(a) providing a cell line according to the present invention;
(b) expressing the glycoprotein of interest in said cell line; and
(c) isolating the glycoprotein from the cell or the cell culture supernatant.

In these aspects, all of the definitions and preferred and/or specific embodiments described for the recombinant glycoproteins of the present invention and the cell lines of the present invention apply in an analogous manner where applicable.

As used herein, the term "about" is intended to be a modifier of ± 10%, preferably ± 5% of the specified value. As an example, the term "about 0.2" is intended to encompass the range of 0.18 to 0.22, preferably 0.19 to 0.21.

The terms "comprising/comprises", "consisting of/consists of", and "consisting essentially of/consists essentially of" are used herein in an interchangeable manner, *i.e*., each of said terms can expressly be exchanged against one of the other two terms.

The figures show:
Figure 1:
   Biosynthesis of tri- and tetra-antennary branched N-glycans. Tri- and tetra-antennary complex N-glycans are generated by the action of GnTlV and GnTV. These branches can be elongated further with e.g. galactose, N-acetylgalactosamine, sialic acid, and fucose.
Figure 2:
   Branch specificity of ST6Gal1 activity. ST6Gal1 catalyzes sialylation of the primary branches of tri- and tetra-antennary N-glycans, whereas sialylation of the additional branches of tri- and tetra-antennary N-glycans is catalyzed by ST3Gal4.
Figure 3:
   DSA and PHA-L lectin immunoblot analysis of N-glycans from human AAT purified from human serum (Prolastin) in comparison to rhAAT expressed in CAP wild-type cells.
Figure 4:
   DSA lectin immunoblot of recombinant hAAT expressed in wildtype CAP cells (control) or CAP cells harboring a GnTlVb knock-out in the presence or absence of sialyltransferases. CAP wild-type cells or CAP cells harboring a GnTlVb knock-out (without (A) or with additional overexpression of sialyltransferases ST3Gal4 (B) or ST6Gal1 (C)) were modified to stably express rhAAT. rhAAT was purified from cell culture supernatants and was subject to lectin blot analysis. *Datura stramonium* (DSA) lectin detects core ß1→4 branched N-glycans synthesized by GnTlVb. Therefore, a diminished signal in the DSA blot means a decreased amount of N-glycans with ß1→4 branches, indicating a decreased or absent GnTlVb enzyme activity, resulting in N-glycan structures without a ß1→4 branch and therefore with only di- or tri-antennary structures. As loading control, the same samples were subject to western blot analysis using a hAAT specific antibody.
Figure 5:
   PHA-L lectin immunoblot of recombinant hAAT expressed in wildtype CAP cells or CAP cells harboring a GnTV knock-out in the presence or absence of sialyltransferases. CAP wild-type cells or CAP cells harboring a GnTV knock-out (with or without additional overexpression of ST6Gal1) were modified in order to stably express rhAAT. rhAAT was purified from cell culture supernatants and was subject to lectin blot analysis. *Phytohemagglutinin*-L (PHA-L) lectin detects core ß1→6 branched N-glycans synthesized by GnTV. Therefore, a diminished signal in the PHA-L blot is indicating a decreased or absent GnTV enzyme activity, resulting in N-glycan structures without a ß1→6 branch and therefore with only di- or tri-antennary structures. Binding of PHA-L lectin to the ß1→6 branched N-glycan is inhibited by sialic acid, therefore signal is decreased in the samples purified from cells additionally expressing ST6Gal1. Nevertheless, a diminished signal in the GnTV knock-out sample in comparison to the CAP wild-type sample can be observed. As loading control the same samples were subject to western blot analysis using an hAAT specific antibody.
Figure 6:
   Summary of MALDI-TOF mass spectrum analysis of rhAAT N-glycans of protein expressed in CAP wild-type or in CAP ΔGnTlVb with or without additional overexpression of ST3Gal4 or ST6GAL1. Displayed are the sums of relative amounts of the hybrid, di-, tri- and tetra-antennary N-glycan structures of CAP ΔGnTlVb normalized to CAP wt. Glycan fragments generated by MALDI-TOF or negligible signal were not used for this analysis. Knock-out of GnTlVb results in a 3- to 15-fold decrease in the total amounts of tetra-antennary N-glycans. Residual amounts of tetra-antennary N-glycans could be due to the enzymatic activity of GnTIVa.
Figure 7:
   DSA and PHA-L lectin immunoblot of recombinant hAAT expressed in wildtype CAP cells or CAP cells harboring a ΔGnTV or ΔGnTlVb/GnTV knock-out. CAP wild-type cells or CAP single cell clones harboring a GnTV, or GnTlVb/GnTV double knock-out were modified in order to stably express rhAAT. rhAAT was purified from cell culture supernatants and was subject to lectin blot analysis. DSA lectin detects core ß1→4 branched N-glycans synthesized by GnTlVb. Therefore, a diminished signal in the DSA blot means a decreased amount of N-glycans with ß1→4 branches. PHA-L lectin detects core ß1→6 branched N-glycans synthesized by GnTV. Therefore, a diminished signal in the PHA-L blot means a decreased amount of N-glycans with ß1→6 branches. AAT purified from CAP single cell clones with the GnTlVb/GnTV double knock-out display only a minor signal in both lectin blot analysis in comparison to the wild type control indicating that almost all N-glycans are di-antennary.
Figure 8:
   Comparison of rhAAT isoform patterns by IEF analysis. As the backbones of the different rhAAT are identical, changes in the IEF are most likely due to changes in the sialic acid content. rhAAT was expressed in wild-type, ΔGnTlVb, ΔGnTV, or ΔGnTlVb/GnTV CAP cells with additional expression of ST6Gal. AAT purified from knock-out cells shows a less acidic pl, indicating a decrease in the total amount of sialic acids per molecule. In addition, this indicates a decrease of potential NeuAc binding sites due to a shift from tri- and tetra-antennary structures to di-antennary N-glycan structures.
Figure 9:
   ECL lectin immunoblot of recombinant hAAT expressed in wildtype CAP cells or CAP cells harboring a ΔGnTlVb/GnTV knock-out. *Erythrina crista-galli* (ECL) lectin detects ß1-4 linked terminal galactose on N-linked glycans. Due to the branch specificity of ST6Gal1 (Gal*ß*1→4GlcNAc*ß*1→2Manα1→3 and Gal*ß*1→4GlcNAc*ß*1→2Manα1→6), overexpression of ST6Gal1 results in complete sialylation of di-antennary N-glycans present on rhAAT expressed in ΔGnTlVb/GnTV cells, whereas rhAAT expressed in wild-type CAP cells with additional overexpression of ST6Gal1 still harbors non-sialylated N-glycan branches. Neuraminidase catalyzes the hydrolysis of N-acetyl-neuraminic acid residues from oligosaccharide, thus neuraminidase treated samples serve as positive control. As loading control, the same samples were subjected to western blot analysis using an hAAT specific antibody.

The present invention will be further illustrated by the following examples without being limited thereto.

### Examples

### Material and Methods:

### Cell lines

Cell lines used in the present application are indicated in Table 1 below.

**Table 1: Stable cell lines used in the present invention.**

| **Cell Line** | **Expression of GnTV/GnTlVb** | **Rec. Protein Expression** | **Overexpression of Sialyltransferases** |
|---|---|---|---|
| CAP | wild-type | rhAAT | |
| CAP | ΔGnTlVb | rhAAT | |
| CAP | Δ-GnTV | rhAAT | |
| CAP | ΔΔGnTlVb/GnTV | rhAAT | |
| CAP | wild-type | rhAAT | ST6Gal1 |
| CAP | ΔGnTlVb | rhAAT | ST6Gal1 |
| CAP | Δ-GnTV | rhAAT | ST6Gal1 |
| CAP | ΔΔGnTlVb/GnTV | rhAAT | ST6Gal1 |
| CAP | wild-type | rhAAT | ST3Gal4 |
| CAP | ΔGnTlVb | rhAAT | ST3Gal4 |

### Cell Culture and Fermentation

The permanent human amniocyte cell line CAP 1 D5 and all their derivates with diminished expression of GnTlVa/b and/or GnTV were cultured in suspension, either in chemically defined, animal component free CAP-CDM medium (CEVEC Pharmaceuticals, Germany) supplemented with 6 mM stable glutamine (biochrom, Germany), or in serum free PEM media (Life Technologies) supplemented with 4 mM stable glutamine (biochrom, Germany).

All cell lines were cultivated at 37 °C in shaker flasks (Corning, #431143, 125 mL (25 mL wv) or #431252, 3000 mL (1000 mL wv)) at 5% CO₂, and 185 rpm. During fermentation CAP cells were fed at d3, d5, and d7 with 10 % CAP-CDM feed solution (CEVEC Pharmaceuticals, Germany) and 4 mM stable glutamine (biochrom, Germany).

### Cloning

For the generation of the CAP cell lines stably expressing ST3Gal4 and/or ST6Gal1, the cells were nucleofected with the corresponding nuclei acid constructs.

For designing the ST3Gal4 cDNA, sequence information of the precursor protein and mature protein was based of the database entry UniProt Q11206. For cloning, a Clal restriction site and a Kozak sequence were added 5' of the start codon of the human ST3Gal4 cDNA and an EcoRV restriction site was added 3' of the stop codon to be inserted between the ClaI and EcoRV restriction sites in the pStbl-Neo-CMV-MCS(-) vector resulting in the expression plasmid pStbl-Neo-CMV-ST3Gal4. This vector contains a CMV promoter driving the expression of the gene of interest, followed by an SV40 intron for improved, splicing-mediated mRNA transport and a multiple cloning site for the insertion of the gene of interest. The selection marker is driven by the human ubiquitin (UbC) promoter. cDNA synthesis was performed at GeneArt (Germany, Life Technologies).

For designing the ST6Gal1 cDNA, sequence information of the precursor protein and mature protein was based of the database entry UniProt P15907. For cloning, a Clal restriction site and a Kozak sequence were added 5' of the start codon of the human ST6Gal1 cDNA and a EcoRV restriction site was added 3' of the stop codon to be inserted between the Clal and EcoRV restriction sites in the pStbl-Neo-CMV-MCS(-) vector resulting in the expression plasmid pStbl-Neo-CMV-ST6Gal1. cDNA synthesis was performed at GeneArt (Germany, Life Technologies).

### Nucleofection and Pool Generation

Nucleofection was performed using a Nucleofector (LONZA) with the appropriate Nucleofector Kit (KitV) according to the manufacturer's protocol. Briefly, during exponential growth phase of the culture 1 × 10⁷ cells were harvested via centrifugation (150 g for 5 min) and resuspended in 100 µl complete nucleofector solution and mixed with a total of 5 µg plasmid. Nucleofection was performed using the X001 program (CAP cells). After the pulse, cells were recovered in 12 ml complete cell culture media in a 125 ml shaking flask. The cells were cultured as before at 37 °C, 5% CO₂, and 185 rpm. 72 to 96 h post-nucleofection cells were selected with 200 µg/ml neomycin in order to generate stable pools.

### Purification of AAT from cell culture supernatants

Following harvest by tangential flow filtration and sterile filtration, the rhAAT containing cell culture supernatant was concentrated and diafiltrated against buffer for the first chromatography step. The diafiltrated solution was loaded on an AAT affinity chromatography column. Subsequently, rhAAT protein was eluted in the linear gradient with increasing MgCl₂ concentrations. The pooled fractions were concentrated and the buffer was exchanged against formulation buffer using desalting columns.

### Lectin Immunoblotting

Lectins are proteins that bind specific carbohydrate structures. Biotin-coupled lectins can therefore be used to analyze N-linked glycans. DSA lectin detects core ß1→4 branched N-glycans synthesized by GnTlVb. PHA-L lectin detects core ß1→6 branched N-glycans synthesized by GnTV. Purified protein or cell culture supernatants with or without diminished expression of GnTIVa/b and/or GnTV and with or without co-expression of ST3Gal4 and/or ST6Gal1 were separated as described above and blotted onto Amersham Hybond ECL nitrocellulose membrane (GE healthcare). The membrane was blocked for 1 h at RT with PBSTB (phosphate-buffered saline, pH = 7.4, supplemented with 0.1 % Tween 20 and 1 % BSA). Afterwards, the membrane was incubated with the lectin diluted 1:2000 in PBSTB. After washing the membrane with PBST (phosphate-buffered saline, pH = 7.4, supplemented with 0.1% Tween 20), the membrane was stained with streptavidin-coupled horseradish peroxidase for 1 h at RT (1:1000 diluted in PBSTB). The HRP signal was amplified using anti-streptavidin IgG and anti IgG-HRP. The proteins were detected using the Pierce ECL WB Substrate Kit via a chemiluminescence detector (INTAS).

### IEF analysis

Isoelectric focusing (IEF) was performed in order to analyze the isoelectric point (pl) of rhAAT purified from CAP cells expressing rhAAT with or without diminished expression of GnTlVa/b and/or GnTV and with or without co-expression of ST3Gal4 and/or ST6Gal1. The degree of sialylation correlates with a given proteins acidity and, therefore, with its pl. IEF analysis was done according to the manufacturers protocol (Invitrogen). Briefly, 5 µg of purified protein were loaded on pH3-7 gels and subjected to electrophoresis (1h 100 V, 1 h 200 V, 30 min 500 V). Proteins were stained with SimplyBlue SafeStain according to the manufacturers protocol (Invitrogen).

### Example 1:

Cells of the human amniocyte cell line CAP 1 D5 were stably transfected with a plasmid coding for rhAAT. After pool generation cells were subject to fed-batch in order to generate sufficient amounts of recombinant AAT.

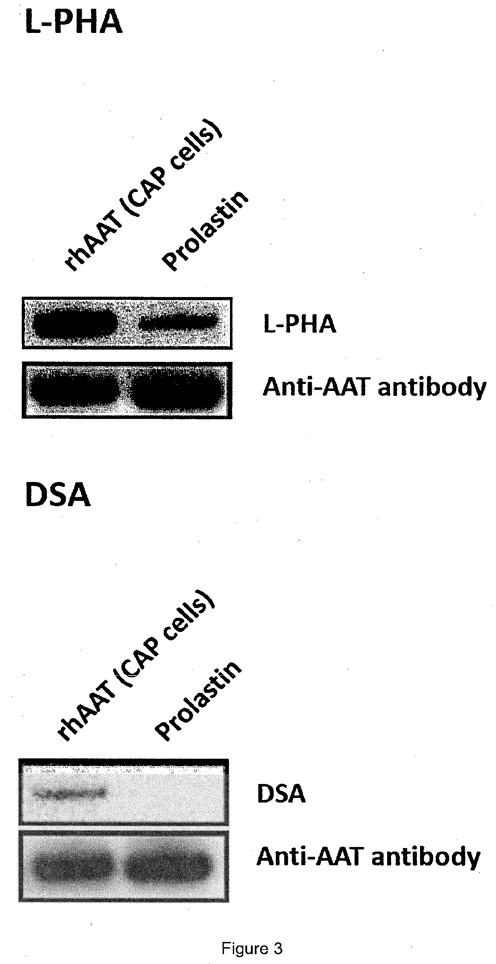

To determine the degree of core ß1,6- and or core ß1,4-branched N-glycans of AAT expressed in wild-type CAP cells in comparison to plasma-derived AAT (Prolastin), DSA and PHA-L lectin blots were performed. PHA-L reacts specifically with core β1,6-branched products synthesized by GnTV and DSA detects core ß1→4 branched N-glycans synthesized by GnTlVb. Therefore, the increased signal in blots shown in Figure 3 indicates a higher degree of tri- and/or tetra-antennary N-glycans in the AAT derived from wild-type CAP cells in comparison to the plasma derived AAT.

Lectin blot analysis (Figure 3) reveals that as in other mammalian cell lines, expression of recombinant glycoproteins results in N-glycan structures with elevated amount of tri- and tetra-antennary structures compared to the corresponding plasma-derived human protein.

### Example 2:

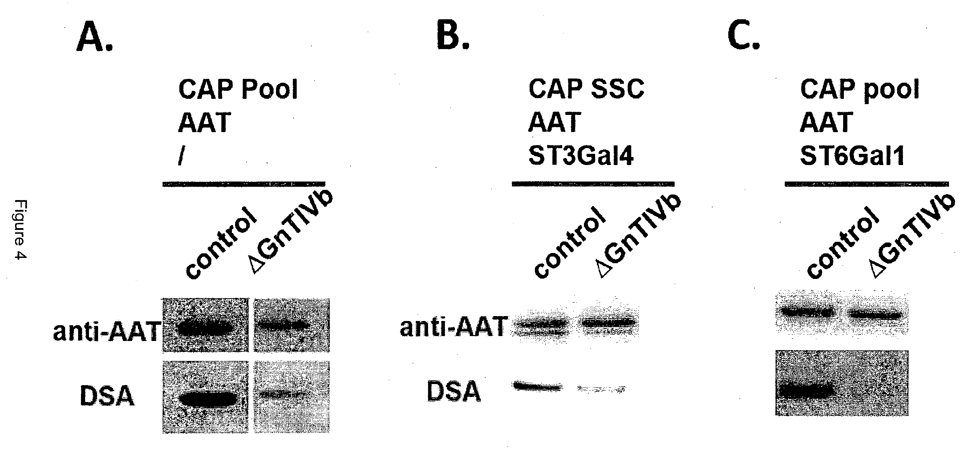

Cells of the human amniocyte cell line CAP 1 D5 with either wild type expression or diminished expression of GnTlVb were stably transfected with a vector encoding rhAAT. After pool generation, cell lines were subject to fed-batch in order to make sufficient amounts of recombinant AAT. rhAAT was purified from cell culture supernatants and was subject to lectin blot analysis. DSA lectin detects core ß1→4 branched N-glycans synthesized by GnTlVb. The reduction in DSA-signal shown in Figure 4A is due to a reduced or absent GnTlVb enzyme activity, resulting in N-glycan structures without a ß1→4 branch and therefore without tetra-antennary structures. As loading control same samples were subject to western blot analysis using a hAAT specific antibody (Figure 4).

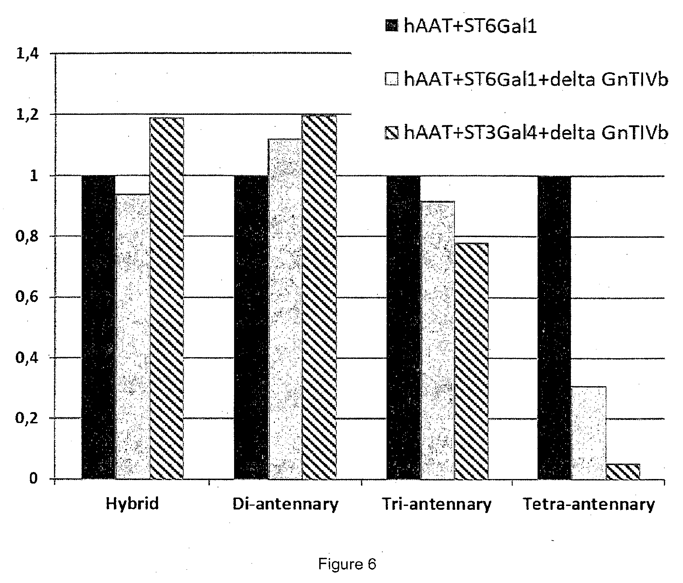

These results were confirmed by MALDI-TOF analysis of N-glycans from AAT derived in CAP wildtype cells and CAP ΔGnTlVb (Figure 6), as knock-out of GnTlVb results in a 3- to 4-fold decrease in the total amounts of tetra-antennary N-glycans. Residual amount of tetra-antennary N-glycan structures are probably due to residual GnTlVb activity in the modified CAP cells or due to GnTIVa enzyme activity.

### Example 3:

Cells of the human amniocyte cell line CAP 1 D5 with either wild type expression or diminished expression of GnTV were stably transfected. After pool generation cell lines were subject to fed-batch in order to make sufficient amounts of recombinant AAT.

Purified recombinant AAT was then analyzed to determine the degree of core ß1,6-branched N-glycans in the AAT expressed in ΔGnTV vs. wild-type CAP cells. In order to do so, a PHA-L (phytohemagglutinin-L) lectin blot was performed. PHA-L reacts specifically with core β1,6-branched glycostructures synthesized by GnTV.

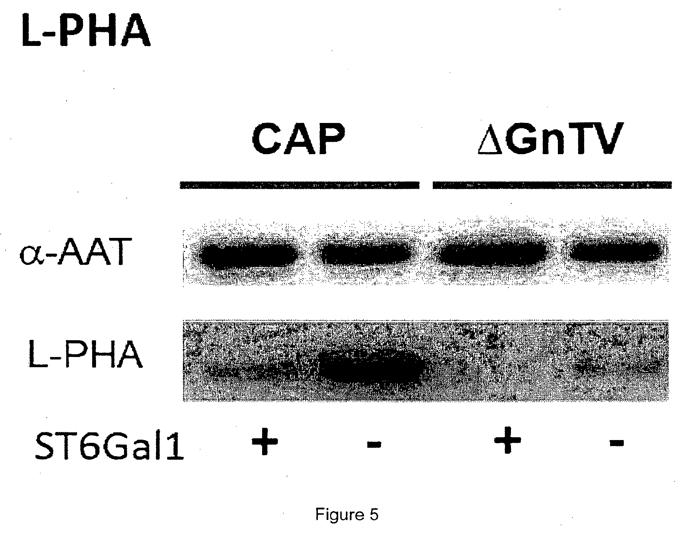

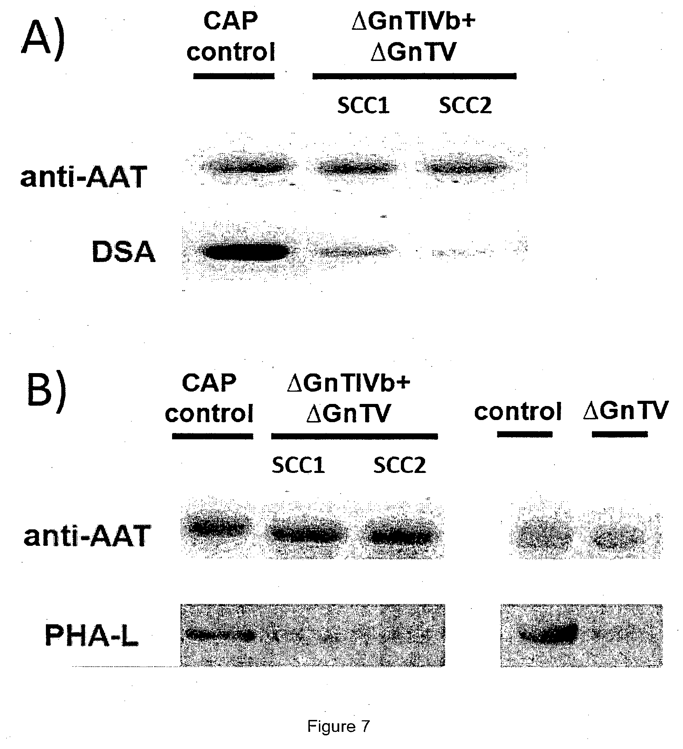

As shown in Figure 5 (lane 2 and 4) and Figure 7B a diminished signal in the PHA-L blot was detected, indicating a decreased or absent GnTV enzyme activity, decreased amount of ß1,6-branched products, and, therefore, reduced amount of tri- and/or tetra-antennary N-glycans.

### Example 4:

In order to investigate if an absent enzyme activity of GnTlVb and GnTV results in only di-antennary N-glycans, the following experiment was performed. CAP cells with either wild type expression or reduced expression of GnTlVb and GnTV (ΔGnTlVb/GnTV cells) were nucleofected with a vector coding for rhAAT. After pool generation cell lines were subject to fed-batch in order to generate sufficient amounts of recombinant AAT. Subsequently, AAT was purified from the cell culture supernatant.

Next, the N-glycan structures from rhAAT expressed in wild-type or ΔGnTlVb/GnTV CAP cells were analyzed by DSA and PHA-L lectin blots (Figure 7).

Interestingly, in the DSA as well as in the PHA-L lectin blots only a background signal was detectable for the rhAAT expressed in ΔGnTlVb/GnTV CAP, strongly suggesting that rhAAT expressed in these cells contains no or only minimal amounts of tri or tetra-antennary N-glycan structures.

### Example 5:

CAP wild-type cells expressing rhAAT or cells with reduced expression of GnTlVb and/or GnTV expressing rhAAT were further transfected with an additional vector encoding for ST6Gal1 or ST3Gal4. After pool generation, generated cell lines were subject to fed-batch in order to generate sufficient amounts of recombinant AAT. Subsequently, AAT was purified from the cell culture supernatant of the following cell lines: CAP-AAT, CAP-AAT+ST6Gal1, CAP-AAT+ST3Gal4, ΔGnTlVb-AAT, ΔGnTlVb-AAT+ST6Gal1, ΔGnTlVb-AAT+ST3Gal4, ΔGnTV-AAT, ΔGnTV-AAT+ST6Gal1, ΔΔGnTlVb/V-AAT, and ΔΔGnTlVb/V-AAT+ST6Gal1.

Figures 4 and 7 show that additional expression of sialyltransferases like ST3Gal4 or ST6Gal1 has no influence on the diminished amount of ß1,6-branched N-glycans caused by the diminished expression of GnTlVb. In addition, Figures 5 and 7 show that additional expression of sialyltransferases has also no influence on the reduced amount of ß1,4-branched N-glycans caused by the diminished expression of GnTV. Of note, binding of PHA-L lectin to the ß1→6 branched N-glycan is inhibited by sialic acid, so that signal is decreased in the samples purified from cells additionally expressing ST6Gal1. Nevertheless, a diminished signal of the GnTV knock-out in comparison to the CAP wild-type cells can be observed.

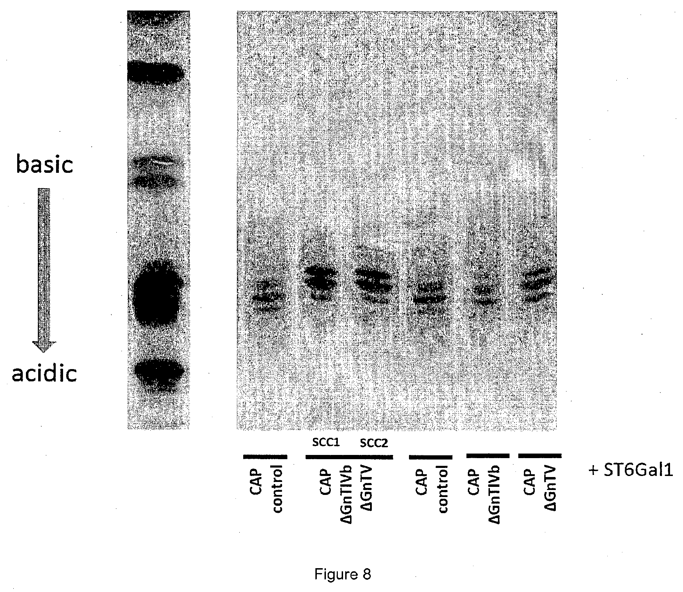

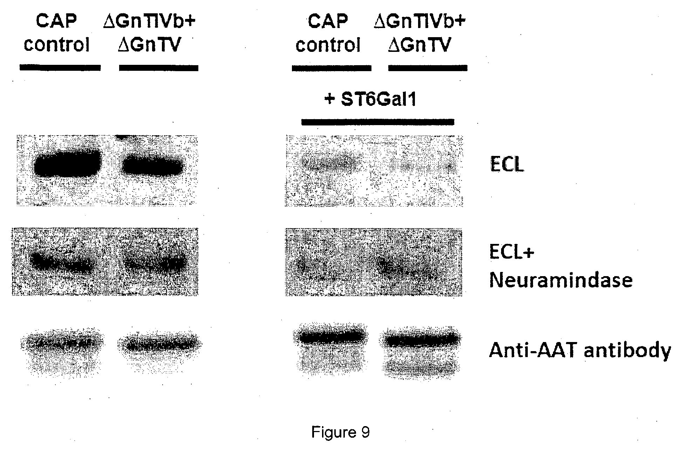

As the backbones of the different rhAAT are identical, changes in the IEF indicate changes in the sialic acid content. rhAAT expressed in ΔGnTlVb, ΔGnTV, or ΔGnTlVb/GnTV CAP cells with additional expression of ST6Gal1 results in a modified rhAAT which slightly shifts towards a less acidic pl, in comparison to the rhAAT expressed in parental CAP cells overexpressing ST6Gal1. This indicates a decrease in the total amount of sialic acids per molecule. The observation is consistent with a decrease of potential NeuAc binding sites due to a shift from tri-and tetra-antennary structures to di-antennary N-glycan structures (Figure 8). This rational is supported by the ECL blot shown in Figure 9.

The present invention discloses the following amino acid sequences.
SEQ ID NO: 1
   Human GnTV 
MALFTPWKLSSQKLGFFLVTFGFIWGMMLLHFTIQQRTQPESSSMLREQILDLSKRYIKA
LAEENRNVVDGPYAGVMTAYDLKKTLAVLLDNILQRIGKLESKVDNLVVNGTGTNSTNST
TAVPSLVALEKINVADIINGAQEKCVLPPMDGYPHCEGKIKWMKDMWRSDPCYADYGVDG
STCSFFIYLSEVENWCPHLPWRAKNPYEEADHNSLAEIRTDFNILYSMMKKHEEFRWMRL
RIRRMADAWIQAIKSLAEKQNLEKRKRKKVLVHLGLLTKESGFKIAETAFSGGPLGELVQ
WSDLITSLYLLGHDIRISASLAELKEIMKKVVGNRSGCPTVGDRIVELIYIDIVGLAQFK
KTLGPSWVHYQCMLRVLDSFGTEPEFNHANYAQSKGHKTPWGKWNLNPQQFYTMFPHTPD
NSFLGFVVEQHLNSSDIHHINEIKRQNQSLVYGKVDSFWKNKKIYLDIIHTYMEVHATVY
GSSTKNIPSYVKNHGILSGRDLQFLLRETKLFVGLGFPYEGPAPLEAIANGCAFLNPKFN
PPKSSKNTDFFIGKPTLRELTSQHPYAEVFIGRPHVWTVDLNNQEEVEDAVKAILNQKIE
PYMPYEFTCEGMLQRINAFIEKQDFCHGQVMWPPLSALQVKLAEPGQSCKQVCQESQLIC
EPSFFQHLNKDKDMLKYKVTCQSSELAKDILVPSFDPKNKHCVFQGDLLLFSCAGAHPRH
QRVCPCRDFIKGQVALCKDCL
SEQ ID NO: 2
   Human GnTlVa 
MRLRNGTVATALAFITSFLTLSWYTTWQNGKEKLIAYQREFLALKERLRIAEHRISQRSS
ELNTIVQQFKRVGAETNGSKDALNKFSDNTLKLLKELTSKKSLQVPSIYYHLPHLLKNEG
SLQPAVQIGNGRTGVSIVMGIPTVKREVKSYLIETLHSLIDNLYPEEKLDCVIVVFIGET
DIDYVHGVVANLEKEFSKEISSGLVEVISPPESYYPDLTNLKETFGDSKERVRWRTKQNL
DYCFLMMYAQEKGIYYIQLEDDIIVKQNYFNTIKNFALQLSSEEWMILEFSQLGFIGKMF
QAPDLTLIVEFIFMFYKEKPIDWLLDHILWVKVCNPEKDAKHCDRQKANLRIRFRPSLFQ
HVGLHSSLSGKIQKLTDKDYMKPLLLKIHVNPPAEVSTSLKVYQGHTLEKTYMGEDFFWA
ITPIAGDYILFKFDKPVNVESYLFHSGNQEHPGDILLNTTVEVLPFKSEGLEISKETKDK
RLEDGYFRIGKFENGVAEGMVDPSLNPISAFRLSVIQNSAVWAILNEIHIKKATN
SEQ ID NO: 3
   Human GnTlVb

MRLRNGTFLTLLLFCLCAFLSLSWYAALSGQKGDVVDVYQREFLALRDRLHAAEQESLKR
SKELNLVLDEIKRAVSERQALRDGDGNRTWGRLTEDPRLKPWNGSHRHVLHLPTVFHHLP
HLLAKESSLQPAVRVGQGRTGVSVVMGIPSVRREVHSYLTDTLHSLISELSPQEKEDSVI
VVLIAETDSQYTSAVTENIKALFPTEIHSGLLEVISPSPHFYPDFSRLRESFGDPKERVR
WRTKQNLDYCFLMMYAQSKGIYYVQLEDDIVAKPNYLSTMKNFALQQPSEDWMILEFSQL
GFIGKMFKSLDLSLIVEFILMFYRDKPIDWLLDHILWVKVCNPEKDAKHCDRQKANLRIR
FKPSLFQHVGTHSSLAGKIQKLKDKDFGKQALRKEHVNPPAEVSTSLKTYQHFTLEKAYL
REDFFWAFTPAAGDFIRFRFFQPLRLERFFFRSGNIEHPEDKLFNTSVEVLPFDNPQSDK
EALQEGRTATLRYPRSPDGYLQIGSFYKGVAEGEVDPAFGPLEALRLSIQTDSPVWVILS
EIFLKKAD

## Claims

1. A cell line that is genetically modified to reduce the expression of at least one of GnTlVa/b and GnTV.

2. The cell line according to claim 1, wherein the expression of all of GnTlVa/b and GnTV is reduced.

3. The cell line according to claim 1 or claim 2, wherein the expression of GnTlVa/b and/or GnTV is completely abolished.

4. The cell line according to any one of claims 1 to 3, wherein the cell line is further genetically modified to overexpress β-galactoside α-2,6-sialyltransferase 1 (ST6Gal1) and/or α-2,3-sialyltransferase 4 (ST3Gal4).

5. The cell line according to any one of claims 1 to 4, wherein the cell line is an insect cell line, an avian cell line, or a mammalian cell line, preferably a human cell line.

6. The cell line according to any one of claims 1 to 5, wherein the cell line is derived from a cell line, selected from the group consisting of Muscovy Duck cells (AGE.CR^{®}), African green monkey kidney epithelial cells (Vero), Madin Darby canine kidney cells (MDCK), baby hamster kidney cells (BHK), Chinese hamster ovary (CHO) cells, human hepatocarcinoma cell lines (HepG2, Huh7), human embryonic kidney 293 (HEK293) cells, human neuronal precursor cells (AGE1.HN^{®}, NC5T11), human embryonic retinoblast cells (Per.C6), myeloma cell lines (HMCLs, MM.1, U266, RPMI8226), CML tumor cell lines (NM, NM-F9), hybrid HEK293 and lymphoma cell (HKB11), and human amniocytes-derived CAP cells.

7. The cell line according to any one of claims 1 to 5, wherein the cell line is derived from human primary amniocytes comprising at least one nucleic acid encoding the gene products of the adenoviral E1 and pIX regions.

8. A recombinant glycoprotein that has been expressed in a cell line according to any one of claims 1 to 7.

9. The recombinant glycoprotein according to claim 8, having N-glycans with a significantly increased proportion of di-antennary N-glycans when compared to the same recombinant glycoprotein expressed in a cell line with non-reduced expression of GnTIVa/b and/or GnTV.

10. The recombinant glycoprotein according to claim 8 or claim 9, wherein the proportion of tetra-antennary N-glycans is significantly reduced when compared to the same recombinant glycoprotein expressed in a cell line with non-reduced expression of GnTIVa/b and/or GnTV.

11. The recombinant glycoprotein according to any one of claims 8 to 10, wherein at least 80% of the antennae of the N-glycans are terminated by 2,6-linked sialic acid.

12. The recombinant glycoprotein according to any one of claims 8 to 11, wherein the glycoprotein is selected from the group consisting of α1-antitrypsin (AAT), hepatocyte growth factor (HGF), Factor VII (FVII), Factor VIII (FVIII), Factor IX (FIX), von-Willebrand-Factor (vWF), and C1 esterase inhibitor (C1-inhibitor; C1 Inh).

13. The recombinant glycoprotein according to any one of claims 8 to 12, wherein the glycoprotein is a mammalian, preferably a human glycoprotein.

14. A method for the expression of a recombinant glycoprotein of interest in a cell, said glycoprotein having N-glycans with a significantly increased proportion of di-antennary N-glycans when compared to the same recombinant glycoprotein expressed in a cell line with non-reduced expression of GnTlVa/b and/or GnTV, comprising the steps of:
(a) providing a cell line according to any one of claim 1 to 8;
(b) expressing the glycoprotein of interest in said cell line; and
(c) isolating the glycoprotein from the cell or the cell culture supernatant.

15. The method according to claim 14, wherein the recombinant glycoprotein is a recombinant glycoprotein according to any one of claims 8 to 13.
